# EUROPEAN PATENT SPECIFICATION

(11) **EP 4 507 690 B1**
(45) Date of publication and mention of the grant of the patent: **27.05.2026**
(21) Application number: 23710777.6
(22) Date of filing: 17.03.2023
(51) Int. Cl.: A61K 31/366, A61K 31/506, A61P 9/00

(54) **COMBINATION OF DASATINIB AND LOVASTATIN FOR USE IN METHODS FOR THE TREATMENT OF CARDIAC FIBROSIS**
KOMBINATION VON DASATINIB UND LOVASTATIN ZUR VERWENDUNG IN VERFAHREN ZUR BEHANDLUNG VON HERZFIBROSE
COMBINAISON DE DASATINIB ET DE LOVASTATIN POUR LEUR UTILISATION DANS LES PROCÉDÉS DE TRAITEMENT DE LA FIBROSE CARDIAQUE

(30) Priority: 18.03.2022 EP 22305324
(43) Date of publication of application: 19.02.2025
(73) Proprietor: INSERM (Institut National de la Santé et de la Recherche Médicale), 75013 Paris (FR); Université Toulouse III - Paul Sabatier, 31400 Toulouse (FR); Centre Hospitalier Universitaire de Toulouse, 31300 Toulouse (FR)
(72) Inventor: KOUNDOUZOVA, Oxana, 31432 TOULOUSE (FR); BOAL, Frédéric, 31432 TOULOUSE (FR); RONCALLI, Jérôme, 31432 TOULOUSE (FR); TRONCHERE, Hélène, 31432 TOULOUSE (FR); PARINI, Angelo, 31432 TOULOUSE (FR); SHARIPOV, Ruslan, 354340 Sochi (FR); KOLPAKOV, Fedor, 354340 Sochi (FR); PINTUS, Sergei, 354340 Sochi (FR); KEL, Alexander, 38302 Wolfenbuettel (DE)
(74) Representative: Inserm Transfert
(86) International application number: PCT/EP2023/056871
(87) International publication number: WO 2023/175136

(56) References cited:
- WO-A1-2016/123086
- WU PEI ET AL: "Lovastatin attenuates angiotensin II induced cardiovascular fibrosis through the suppression of YAP/TAZ signaling", BIOCHEMICAL AND BIOPHYSICAL RESEARCH COMMUNICATIONS, vol. 512, no. 4, 14 May 2019 (2019-05-14), pages 736 - 741, XP085659262, ISSN: 0006-291X, DOI: 10.1016/J.BBRC.2019.03.158
- HUELSENBECK J ET AL: "Inhibition of Rac1 signaling by lovastatin protects against anthracycline-induced cardiac toxicity", vol. 2, no. 8, 1 August 2011 (2011-08-01), pages e190 - e190, XP055824559, Retrieved from the Internet <URL:https://www.ncbi.nlm.nih.gov/pmc/articles/PMC3181415/pdf/cddis201165a.pdf> DOI: 10.1038/cddis.2011.65
- MEYER-TER-VEHN TOBIAS ET AL: "Lovastatin Inhibits TGF-??Induced Myofibroblast Transdifferentiation in Human Tenon Fibroblasts", INVESTIGATIVE OPTHALMOLOGY & VISUAL SCIENCE, vol. 49, no. 9, 1 September 2008 (2008-09-01), US, pages 3955, XP055824554, ISSN: 1552-5783, DOI: 10.1167/iovs.07-1610
- MA ZEJUN ET AL: "Lovastatin Alleviates Endothelial-to-Mesenchymal Transition in Glomeruli via Suppression of Oxidative Stress and TGF-?1 Signaling", FRONTIERS IN PHARMACOLOGY, vol. 8, 18 July 2017 (2017-07-18), XP055824561, DOI: 10.3389/fphar.2017.00473
- BALASUBRAMANIAN SUNDARAVADIVEL ET AL: "Dasatinib Attenuates Pressure Overload Induced Cardiac Fibrosis in a Murine Transverse Aortic Constriction Model", vol. 10, no. 10, 12 October 2015 (2015-10-12), pages e0140273, XP055824799, Retrieved from the Internet <URL:https://www.ncbi.nlm.nih.gov/pmc/articles/PMC4601773/pdf/pone.0140273.pdf> DOI: 10.1371/journal.pone.0140273

## Description

### FIELD OF THE INVENTION:

The present invention is in the field of medicine, in particular cardiology.

### BACKGROUND OF THE INVENTION:

Cardiac fibrosis represents a major unresolved medical challenge resulting in significant morbidity and mortality [1]. Fibrosis is defined as the replacement of healthy tissue by an excessive deposition of extracellular matrix (ECM), leading progressively to the loss of the original tissue architecture and function [2]. The initial cardiac fibrotic remodeling can result from multiple acute or chronic stimuli, including mechanical injury, chronic inflammation, autoimmune reactions or infections [3]. Under the heart disease umbrella, the extent of myocardial fibrosis correlates with mortality and the incidence of serious cardiovascular side effects in heart failure (HF) patients [4-6]. Hence, the lack of effective anti-fibrosis therapies and inadequate understanding of the molecular pathogenesis of fibrotic remodeling represents an unmet and urgent clinical need.

Fibrotic remodeling is a complex and coordinated machinery triggering a complex cascade of molecular responses that culminates in phenotypic reprogramming of the heart [2,7]. Uncontrolled fibroblast transition from naive to activated state is a central element dictating resolution of organ fibrosis [8]. In the heart, fibroblasts represent not only a predominant cell type, but also a major source of the cellular tissue microenvironment comprising the ECM and soluble factors. A key step in the activation of fibrotic remodeling is the transformation of fibroblasts to α-smooth muscle actin (SMA)-positive myofibroblasts [9] leading to excessive ECM deposition and destruction of normal tissue architecture, cardiac dysfunction and failure [1]. Although the molecular mechanisms of persistent fibroblast activation have not been elucidated, transforming growth factor-β (TGF-β) is considered a cardinal driver of fibroblast-myofibroblast transformation leading to fibrosis development and progression [10]. Depiction of the mechanisms orchestrating the activation of fibroblasts is an essential step towards establishing new therapeutic strategies to combat fibrotic remodeling and organ failure. A cardiac fibrosis-reducing effect was known for lovastatin from Huelsenbeck J et al: Cell Death and Disease (2011) 2, e190 and for dasatinib from Sundaravadivel Balasubramanian et al: PLOS ONE (2015) 10, no. 10, e0140273.

### SUMMARY OF THE INVENTION:

The present invention is defined by the claims. In particular, the present invention relates to a combination of dasatinib and lovastatin for use in methods of treating cardiac fibrosis.

### DETAILED DESCRIPTION OF THE INVENTION:

Cardiac fibrosis is a key component in the pathogenesis of heart disease and represents an urgent unmet clinical need due to the lack of effective therapies. In this study, the Inventors investigated the effect of the combination of lovastatin with dasatinib on collagen production and mitochondrial ROS generation. They demonstrated that lovastatin and dasatinib had synergistic antioxidant effects in human cardiac fibroblasts. Furthermore, the combined treatments of fibroblasts significantly reduced mitochondrial ROS production. Overall, the results show that the combination therapy with lovastatin and dasatinib is suitable for the treatment of cardiac fibrosis.

The first object of the present invention relates to a combination of dasatinib and lovastatin for use in a method of treating cardiac fibrosis in a patient in need thereof comprising administering to the patient a therapeutically effective combination of dasatinib and lovastatin.

As used herein, the term "**cardiac fibrosis**" has its general meaning in the art and refers to a condition characterized by the excessive production and deposition of ECM proteins into the myocardium which leads to normal tissue architecture disruption, reduced tissue compliance, mechanical and electrical dysfunction. Cardiac fibrosis arises from exposure to certain drugs, or in response to various heart diseases, such as myocardial infarction and hypertension. Following acute myocardial infarction, sudden loss of a large number of cardiomyocytes triggers an inflammatory reaction, ultimately leading to replacement of dead myocardium with a collagen-based scar. Several other pathophysiologic conditions induce more insidious interstitial and perivascular deposition of collagen, in the absence of completed infarction. Aging is associated with progressive fibrosis that may contribute to the development of diastolic HF in elderly patients. Pressure overload, induced by hypertension or aortic stenosis, results in extensive cardiac fibrosis that is initially associated with increased stiffness and diastolic dysfunction. Volume overload due to valvular regurgitant lesions may also result in cardiac fibrosis. Hypertrophic cardiomyopathy and post-viral dilated cardiomyopathy are also often associated with the development of significant cardiac fibrosis. Moreover, a variety of toxic insults (such as alcohol or anthracyclines) and metabolic disturbances (such as diabetes and obesity) induce cardiac fibrosis.

As used herein, the term "**treatment**" or "**treat**" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein, the term "**dasatinib**" has its general meaning in the art and refers N-(2-chloro-6-methylphenyl)-2-[[6-[4-(2-hydroxyethyl)-1-piperazinyl]-2-methyl-4-pyrimidinyl]amino]-5-thiazole carboxamide monohydrate. Dasatinib is an ATP-competitive protein tyrosine-kinase inhibitor (CAS N°302962-49-8).

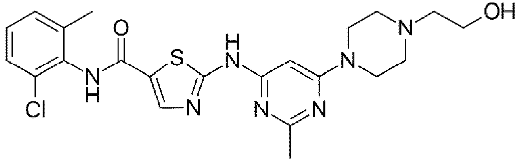

As used herein, the term "**lovastatin**" has its general meaning in the art and refers to [8-[2-(4-hydroxy-6-oxo-oxan-2-yl)ethyl] -3,7-dimethyl-1,2,3,7,8,8a- hexahydronaphthalen- 1-yl] 2-methylbutanoate. Lovastatin is an inhibitor of 3-hydroxy-3-methylglutaryl-coenzyme A reductase (HMG-CoA reductase), an enzyme that catalyzes the conversion of HMG-CoA to mevalonate (CAS N°75330-75-5).

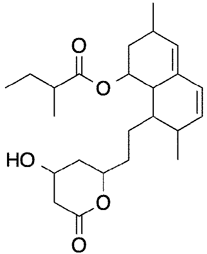

As used herein, the term "**combination**" is intended to refer to all forms of administration that provide a first drug together with a further (second, third...) drug. The drugs may be administered simultaneous, separate or sequential and in any order. The term "**simultaneous**" refers to the use of at least two active ingredients occurring at the same time or at substantially the same time. The term "**separate**" refers to the use of at least two active ingredients not occurring at the same time. The term "**sequential**" refers to the use of at least two active ingredients occurring by following an order. Drugs administered in combination have biological activity in the subject to which the drugs are delivered. Within the context of the invention, a combination thus comprises at least two different drugs, and wherein one drug is dasatinib and wherein the other drug is lovastatin.

In particular, the combination dasatinib with lovastatin results in a reduced fibrosis progression.

In particular, the combination of dasatinib with lovastatin results in synergistic effects as demonstrated in the EXAMPLE. As used herein, the term "**synergistic effect**" to action of two therapeutic agents such as, for example, (a) dasatinib, and (b) lovastatin, producing an effect, for example, reducing the progression of fibrosis, which is greater than the simple addition of the effects of each drug administered by themselves. A synergistic effect can be calculated using suitable methods such as described in the EXAMPLE. In particular, the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively. Synergy may be further shown by calculating the synergy score of the combination according to methods known by one of ordinary skill. The synergy can be assessed with the MacSynergy II (Prichard, M.N. and C. Shipman, Jr. 1990. A three-dimensional model to analyze drug-drug interactions. Antiviral Res. 14:181-206). This program allows the three-dimensional examination of drug interactions of all data points generated from the checkerboard combination of two inhibitors with Bliss- Independence model. Confidence bounds are determined from replicate data. If the 95% confidence limits (CL) do not overlap the theoretic additive surface, then the interaction between the two drugs differs significantly from additive. The volumes of synergy or antagonism can be determined and graphically depicted in three dimensions and represent the relative quantity of synergism or antagonism per change in the two drug concentrations. Synergy and antagonism volumes are based on the Bliss independence model, which assumes that both compounds act independently on different targets. A set of predicted fractional responses faAB under the Bliss independence model is calculated as faAB =faA +faB - faA · faB with faA and faB representing the fraction of possible responses, e.g. % inhibition, of compounds A and B at amounts dA and dB, respectively, and describes the % inhibition of a combination of compounds A and B at amount (dA+dB). If faAB>faA + faB - faA · faB then we have Bliss synergy; if faAB<faA+ faB - faA · faB then we have Bliss antagonism. The 95% synergy/antagonism volumes are the summation of the differences between the observed inhibition and the 95% confidence limit on the prediction of faAB under the Bliss independence model.

As used herein, the term "**therapeutically effective amount**" is meant a sufficient amount of the drug for treating or reducing the symptoms at reasonable benefit/risk ratio applicable to any medical treatment. It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination with the drugs; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the drug for the symptomatic adjustment of the dosage to the subject to be treated. A medicament typically contains from about 0.01 mg to about 500 mg of the drug, typically from 1 mg to about 100 mg of the drug. An effective amount of the drug is ordinarily supplied at a dosage level from 0.0002 mg/kg to about 20 mg/kg of body weight per day, especially from about 0.001 mg/kg to 7 mg/kg of body weight per day.

Typically, the drug of the present invention is administered to the subject in the form of a pharmaceutical composition which comprises a pharmaceutically acceptable carrier. Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a subject, the composition will be formulated for administration to the subject. The compositions of the present invention may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. For example, an antibody present in a pharmaceutical composition of this invention can be supplied at a concentration of 10 mg/mL in either 100 mg (10 mL) or 500 mg (50 mL) single-use vials. The product is formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. An exemplary suitable dosage range for an antibody in a pharmaceutical composition of this invention may between about 1 mg/m² and 500 mg/m². However, it will be appreciated that these schedules are exemplary and that an optimal schedule and regimen can be adapted taking into account the affinity and tolerability of the particular antibody in the pharmaceutical composition that must be determined in clinical trials. A pharmaceutical composition of the invention for injection (e.g., intramuscular, i.v.) could be prepared to contain sterile buffered water (e.g. 1 ml for intramuscular), and between about 1 ng to about 100 mg, e.g. about 50 ng to about 30 mg or more preferably, about 5 mg to about 25 mg, of the inhibitor of the invention.

The invention will be further illustrated by the following figures and examples. However, these examples and figures should not be interpreted in any way as limiting the scope of the present invention.

### FIGURES:

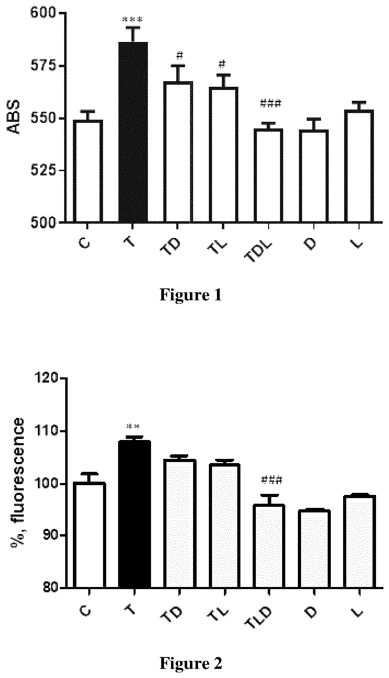

**Figure 1****. Synergic effects of lovastatin and dasatinib on TGFβ-induced collagen production in human cardiac fibroblasts.** Quantitative analysis of cardiac fibroblasts treated with 5 µM lovastatin (L) or 5nM dasatinib (D) or combination of 5 µM lovastatin and 5nM dasatinib (DL), exposed to 10 ng/ml TGFβ (T) for 72 h and stained with Sirius Red. Data are the mean ± SEM of three independent experiments. ****P*< 0.001 vs Control (C); *#P*<0.05; *###P*<0.001 vs TGFβ.
**Figure 2****. Synergic effects of lovastatin and dasatinib on TGFβ-induced mitochondrial ROS production in human cardiac fibroblasts.** Quantification of MitoSox red staining in cardiac fibroblasts exposed to 10 ng/ml TGFβ (T) for 72h in the presence or absence of 5 µM lovastatin (L) or 5nM dasatinib (D) or combination of 5 µM lovastatin and 5nM dasatinib (DL). Data are the mean ± SEM of three independent experiments. ***P*< 0.01 vs Control (C); ###*P*<0.001 vs TGFβ.

### EXAMPLE:

### Methods

### Cell culture and treatments

Primary cardiac fibroblasts were isolated and cultured according to the method described previously [11]. Briefly, the heart was isolated from adult male C57BL6/J mice, triturated and was then incubated with Digestion Buffer [HBSS (Sigma H8264); BSA (1 mg/mL); Pancreatin NB (Serva #31442; 0.5 mg/mL) and Collagenase NB4 (Serva #17454.01; 0.1 mg/mL)] at 37°C for four consecutive rounds of digestion. The suspension was centrifuged at 300 g for 5 min and the cell pellet was resuspended in DMEM F12 medium (Sigma D6434) supplemented with 10% FBS, penicillin (20 U/mL)-streptomycin (20 µg/mL) and 2 mM glutamine and cultured in a 37°C, 5% CO₂ incubator. Primary cardiac fibroblasts were used up to passage 3. Cardiac fibroblasts were exposed to 10 ng/ml TGFβ (T) for 72h in the presence or absence of 5 µM lovastatin (L) or 5nM dasatinib (D) or combination of 5 µM lovastatin and 5nM dasatinib (DL).

### Picrosirius red staining

Following treatment, cardiac fibroblasts were fixed in methanol, and incubated in the 0.1% Picrosirius red staining solution (Sigma-Aldrich, France) as per manufacturer's instructions. Picrosirius red was solubilised in 0.1 N sodium hydroxide and the optical density was read at 540 nm (SERLABO Technologies, France).

### Evaluation of ROS production

Mitochondrial superoxide levels on heart cryosections and cells were assessed with MitoSOX Red fluorescent probe (Life Technologies) and imaged by confocal microscopy as described [12].

### Statistical analysis

Data are expressed as mean±SEM. Comparison between two groups was performed by Student's two-tailed t-test while comparison of multiple groups was performed by one-way ANOVA followed by a Bonferroni's post hoc test using GraphPad Prism version 5.00 (GraphPad Software, Inc).

### Results

To test potential synergistic activity, we investigated the effect of combined treatment with lovastatin and dasatinib on collagen synthesis. As shown in **Figure 1**, low-dose treatment of cardiac fibroblasts with lovastatin (5µM) or dasatinib (5nM) for 72h showed modest but significant effects (p<0.05) on TGFβ-induced collagen production. Consequently, these values did not reach control values in lovastatin- or dasatinib-treated cells. However, combined treatments of fibroblasts with lovastatin (5µM) and dasatinib (5nM) show highly suppressive effect on collagen synthesis in fibroblasts exposed to 10 ng/ml TGFβ (p<0.001). Importantly, within 72 h of exposure to TGFβ, combined treatment with lovastatin and dasatinib maintained collagen content at a value comparable to control level. We further demonstrated that lovastatin (5µM) and dasatinib (5nM) exhibit synergism in reducing mitochondrial ROS production in cell exposed to 10 ng/ml TGFβ for 72h (**Figure 2**),

### REFERENCES:

Throughout this application, various references describe the state of the art to which this invention pertains.
1. Gyöngyösi M, Winkler J, Ramos I, Do Q-T, Firat H, McDonald K, et al. Myocardial fibrosis: biomedical research from bench to bedside. Eur J Heart Fail. 2017;19(2):177-191.doi:10.1002/ejhf.696.
2. Travers JG, Kamal FA, Robbins J, Yutzey KE, Blaxall BC. Cardiac fibrosis: The fibroblast awakens. Circ Res. 2016;118(6):1021-40.doi:10.1161/CIRCRESAHA.115.306565.
3. Baues M, Dasgupta A, Ehling J, Prakash J, Boor P, Tacke F, et al. Fibrosis imaging: Current concepts and future directions. Adv Drug Deliv Rev. 2017;121:9-26.doi:10.1016/j.addr.2017.10.013.
4. Assomull RG, Prasad SK, Lyne J, Smith G, Burman ED, Khan M, et al. Cardiovascular magnetic resonance, fibrosis, and prognosis in dilated cardiomyopathy. J Am Coll Cardiol. 2006;48(10):1977-85.doi:10.1016/j.jacc.2006.07.049.
5. Aoki T, Fukumoto Y, Sugimura K, Oikawa M, Satoh K, Nakano M, et al. Prognostic impact of myocardial interstitial fibrosis in non-Ischemic heart failure. Circ J. 2011;75(11):2605-13. doi:10.1253/circj.cj-11-0568.
6. Gulati A,Jabbour A, Ismail TF, Guha K, Khwaja J, Raza S, et al. Association of fibrosis with mortality and sudden cardiac death in patients with non-ischemic dilated cardiomyopathy. JAMA. 2013;309(9):896-908. doi:10.1001/jama.2013.1363.
7. Bektik E, Fu J. Ameliorating the Fibrotic Remodeling of the Heart through Direct Cardiac Reprogramming. Cells. 2019;8(7):679.doi:10.3390/cells8070679 .
8. Gourdie RG, Dimmeler S, Kohl P. Novel therapeutic strategies targeting fibroblasts and fibrosis in heart disease. Nat Rev Drug Discov. 2016;15(9):620-638. doi: 10.1038/nrd.2016.89.
9. Goldsmith EC, Bradshaw AD, Zile MR, Spinale FG. Myocardial fibroblast-matrix interactions and potential therapeutic targets. J Mol Cell Cardiol. 2014;70:92-9.doi:10.1016/j.yjmcc.2014.01.008.
10. Humeres C, Frangogiannis NG. Fibroblasts in the Infarcted, Remodeling, and Failing Heart. JACC Basic TranslSci. 2019;4(3):449-467. doi:10.1016/j.jacbts.2019.02.006.
11. Pchejetski D, Foussal C, Alfarano C, Lairez O, Calise D, Guilbeau-Frugier C. et al. Apelin prevents cardiac fibroblast activation and collagen production through inhibition of sphingosine kinase 1. Eur Hear J. 2012;33:2360-9
12. H. Tronchere, M. Cinato, A. Timotin, L. Guitou, C. Villedieu, H. Thibault, et al. Inhibition of PIKfyve prevents myocardial apoptosis and hypertrophy through activation of SIRT3 in obese mice. EMBO Mol Med., 9 (2017), pp. 770-785

## Claims

1. A combination of dasatinib and lovastatin for use in a method of treating cardiac fibrosis in a patient in need thereof.

2. The combination for use according to claim 1 wherein the cardiac fibrosis arises from exposure to certain drugs, or in response to various heart diseases, such as myocardial infarction and hypertension.

## Patentansprüche

1. Kombination aus Dasatinib und Lovastatin zur Verwendung in einem Verfahren zur Behandlung von Herzfibrose bei einem Patienten, der diese benötigt.

2. Kombination zur Verwendung nach Anspruch 1, wobei die Herzfibrose durch die Exposition gegenüber bestimmten Arzneimitteln oder als Reaktion auf verschiedene Herzkrankheiten wie Myokardinfarkt und Bluthochdruck entsteht.

## Revendications

1. Combinaison de dasatinib et de lovastatine pour une utilisation dans un procédé de traitement de la fibrose cardiaque chez un patient qui en a besoin.

2. Combinaison pour une utilisation selon la revendication 1 dans laquelle la fibrose cardiaque survient suite à l'exposition à certains médicaments ou en réponse à diverses maladies cardiaques, telles que l'infarctus du myocarde et l'hypertension.
